Europäisches Patentamt

European Patent Office

Office européen des brevets

(11) Publication number: **0 148 586**
**A1**

(12)

# EUROPEAN PATENT APPLICATION

(21) Application number: **84308365.0**

(22) Date of filing: **03.12.84**

(51) Int. Cl.⁴: **A 61 F 5/42**

(30) Priority: **12.12.83 US 560194**

(43) Date of publication of application: **17.07.85**
**Bulletin 85/29**

(84) Designated Contracting States: **AT BE CH DE FR GB IT LI LU NL SE**

(71) Applicant: **Nadig, Perry W., 111 Cotillion, San Antonio Texas 78213 (US)**
Applicant: **Burgeson, Duane A., 10332 Rempas Ct., Albuquerque New Mexico 87114 (US)**

(72) Inventor: **Nadig, Perry W., 111 Cotillion, San Antonio Texas 78213 (US)**
Inventor: **Burgeson, Duane A., 10332 Rempas Ct., Albuquerque New Mexico 87114 (US)**

(74) Representative: **McCallum, William Potter et al, Cruikshank & Fairweather 19 Royal Exchange Square, Glasgow G1 3AE Scotland (GB)**

(54) **Erection apparatus and method.**

(57) An apparatus and method for inducing an erection of a male genital organ having an elongated tubular means (10) with open and closed ends (12, 11) for receiving a male genital organ and means for connecting a vacuum source (24) with the tubular means (10) for inducing an erection of the male genital organ, the closed end (11) having a removable end closure and support means (13) for cleaning of the apparatus and for engaging a support surface to assist in using the apparatus and the open end (12) having a removable insert means (21, 21') for effecting a seal between the user's organ and the tubular means (10) for inducing an erection.

## ERECTION APPARATUS AND METHOD

This invention relates generally to a therapeutic medical device of assisting and obtaining erection and distention of the male genital organ by inducing blood flow into the organ. More particularly the invention relates to an apparatus and method for simulation of an erection of the genital organ of an impotent male.

It is known in the prior art that an erection of the genital organ of the male can be induced by causing blood flow into the organ and obstructing the venous return from that organ to facilitate and enhance penal erection. Known U.S. Patents relating to this area are as follows:

| U.S. Patent Number | Inventor |
|---|---|
| DES 258,690 | An C. Wu |
| 594,815 | H.D. Taggart |
| 1,225,341 | O. Lederer |
| 2,874,698 | F.W. Sell |
| 3,455,301 | B.P. Clark |
| 3,496,589 | J.C. Clement |
| 3,744,486 | E.M. Wilson |
| 3,910,262 | D.T. Stoughton |
| 4,139,007 | H. Diamond |
| 4,175,554 | F.J. Gerow |
| 4,378,008 | G.D. Osbon, Sr. |

| Foreign Patent Number | Inventor |
|---|---|
| 313,836/Italian | Josef Dellner |
| 347,300/Swiss | Giuseppe Meldi |
| 574,684/German | Arthur Skora |

Other known devices which assist in obtaining simulating and/or maintaining an erection include an elongated rigid or semi-rigid sleeve which is positioned along the surface of the male genital organ to provide rigidity and the implantation of a rigid member in the cavity of the male genital organ and the implantation of an inflatable member in the cavity of the organ. The implantations do not induce the flow of blood into the organ to stiffen it, but rather provide stiffening through its own rigidity or by inflation of the implant.

Several of the above devices include a tubular vacuum chamber which is positioned over the flaccid male genital organ for use. A vacuum is then applied to the device by various means and assuming a sufficient seal is effected between the base of the organ and the device this will induce the flow of blood into the organ. After a flow of blood into the organ sufficient to cause an erection is achieved, a restriction member is then placed at the base of the organ to retain the blood therein and maintain the organ in its erect state for a period of time. Typically, an elastic band is fitted over the base of the organ which restricts the flow of blood out of the organ, after release of the vacuum, to maintain the erection.

The above listed patents to Lederer, Sell, Wilson, Gerow and Osbon, utilize a vacuum cylinder for assisting an erection of the male genital organ. All of these patents except Gerow also use a restriction device to maintain the erection after the removal of the vacuum cylinder. As will be apparent, the devices require some training or dexterity to use. Since it may not be desirable or possible to have someone assist in the use it is preferable to provide a device which can be used only by the male without any assistance. Furthermore, the vacuum source, such as a vacuum pump may not be always available or convenient. One alternative is to apply the vacuum with a hand pump of some type such as shown in the patents. Another known type of method for inducing a vacuum in the vacuum cylinder is to connect a tube thereto which extends to the user's mouth so that he can apply a suction force to the tube which will in turn create a vacuum within the vacuum cylinder. In this situation, both hands of the user may not be available to position the vacuum cylinder to create a seal between the base of the male genital organ and the vacuum cylinder.   Also, it is desirable to provide a device which can accommodate varying sizes of genital organs in the flaccid and erect states.   Generally a lubricant is necessary to facilitate insertion and removal of the genital organ.   For this reason it is desirable to provide a vacuum cylinder which can be easily cleaned and maintained in a sanitary condition for repeated use.

It is an object of the present invention to obviate or mitigate the shortcomings and difficulties of prior art devices.

According to the present invention there is provided an appartus for inducing an erection of a male genital organ, comprising:

an elongated tubular means having open and closed ends for receiving a male genital organ in the open end;

means for connecting a vacuum source with the tubular means for inducing an erection of the organ;

said closed end having removable end closure and support means for cleaning the tubular means and which is configured for engaging a support surface with the tubular means to assist the user in holding and supporting the tubular means in position on the user's organ for inducing an erection; and

said open end having a removable insert means to maintain a vacuum in the tubular means for inducing an erection.

Embodiments of the present invention will now be described, by way of example, with reference to the accompanying drawings in which:-

Fig. 1 shows an exploded perspective view of an embodiment of the apparatus of the invention.

Fig. 2 shows a cross-sectional view of the apparatus of the invention with the genital organ in the flaccid state and the end of the vacuum chamber positioned against a vertical rigid object to effect the seal between an insert and the base of the genital organ.

Fig. 3 shows a cross-sectional view of the apparatus of the invention after a genital organ has been induced to an erect state.

Fig. 4 shows the vacuum chamber removed from the insert after achieving erection.

Fig. 5 is a cross-sectional view showing an automatic and manually activated relief valve which limits the amount of vacuum which may be applied to the vacuum chamber and which releases the vacuum when the user so desires.

Fig. 6 shows a cross-sectional view of another embodiment of the vacuum chamber apparatus.

Fig. 7 shows a cross-sectional view taken along line 7-7 of Fig. 6.

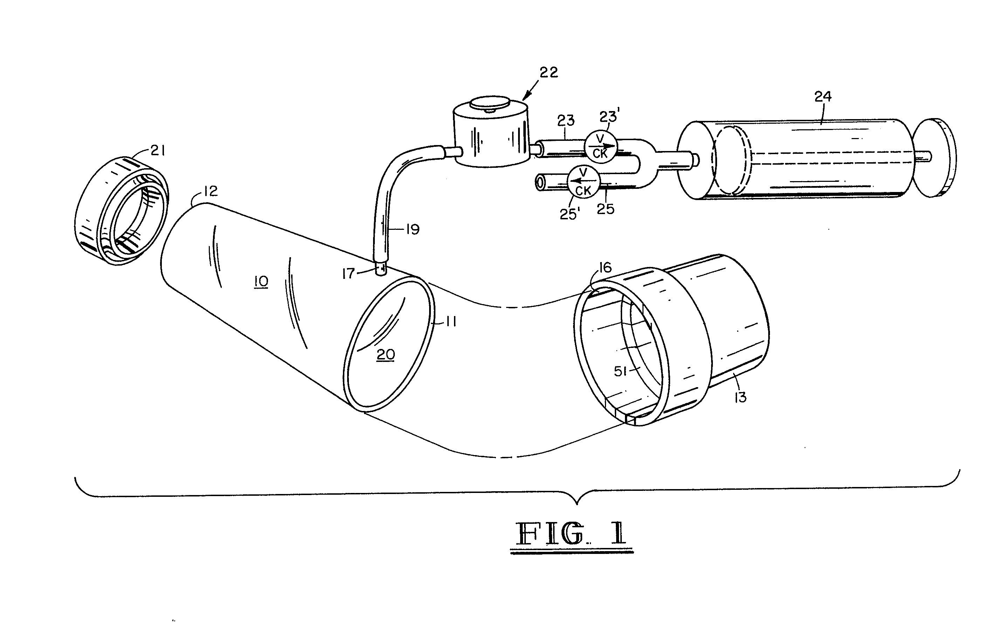

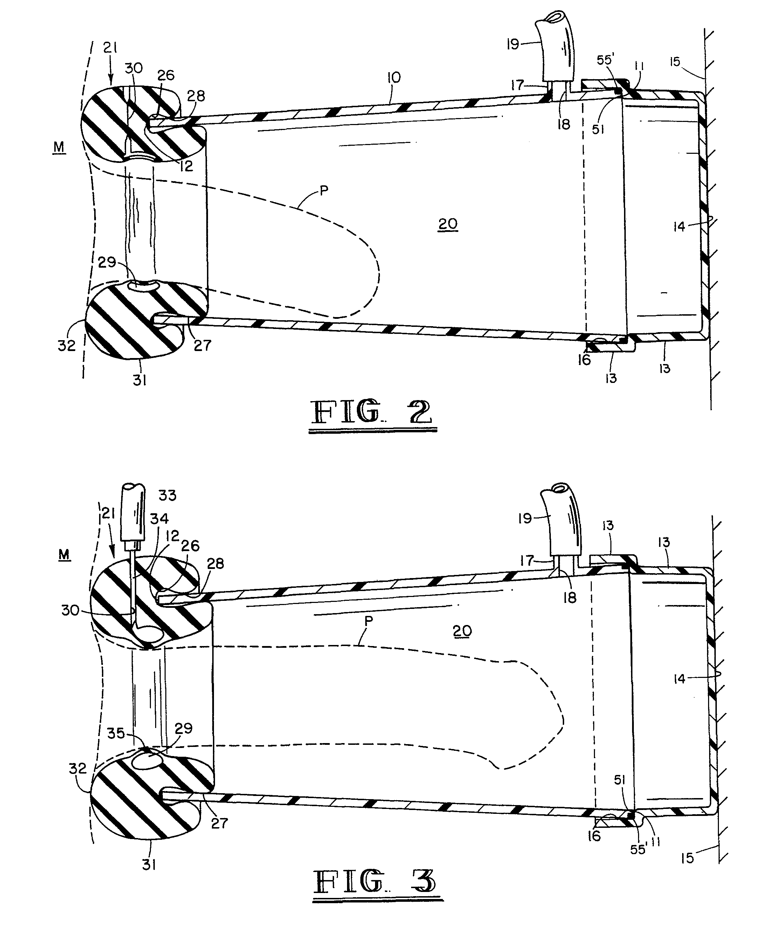

A first embodiment of the invention is shown in Fig. 1 and 2. The apparatus includes a transparent elongated tubular member 10 having a generally circular cross section which forms a portion of the vacuum chamber. The tubular chamber 10 tapers or reduces in diameter from its distal end 11 to its proximal end 12. A removable end cap 13 is releasably positioned on the distal end 11 and forms a seal with distal end 11 at their point of connection. The outer surface 14 of the end cap is flat or so configured that it may be engaged against a suitable force resisting surface such as a wall 15 and easily retained in this engaging position.

As shown in Figs. 1, 2, 3 and 6 the elongated tubular member 10 has a distal end 11 and a proximal end 12. The removable end cap 13 is mounted on the distal end 11 and includes a configured outer surface 14. The tubular member 10 tapers from end 11 to end 12 and has a generally circular cross section. As will be apparent, the distal end 11 is of the greater diameter and the tubular member 10 tapers to the smaller diameter of the proximal end 12. The tubular member 10 includes a nipple 17 connecting with an aperture 18 for a connection with a

0148586

tube which is connected to a vacuum source. The nipple 17 may also be located on the side of end cap 13 in case the user's organ achieves an erect size such that it might extend past the nipple.

The distal end 11 engages the surface 51 on the end cap 13 to form a seal when the removable end cap member 13 is positioned on the tubular member 10. A plurality of alignment raised portions 52, 53 and 54 help position the removal end cap 13 so that a seal is effected and enhanced between the surfaces 11 and 51. A notch or removed portion 55 in the distal end 11 receives a seal ring 55' to help effect a seal between the surfaces 11 and 51.

A nipple extension member 17 is integrally mounted on the tubular member 10 or side of end cap 13 and connects with an aperture 18 in the tubular member 10. A tube 19 is force fitted over the nipple member 17 to permit introducing a vacuum within the vacuum chamber 20 formed by the tubular member 10, end cap 13 and insert 21. A safety release valve 22 is connected with the tube 19 and another tube 23 is connected with the safety release valve 22. An air evacuation device 24 which may be a large syringe is connected to the tube 23. Tube 23 includes a one-way check valve 23' that allows air to flow only from chamber 20. Tube 25 includes another one-way check valve 25' which allows air to pass out of the evacuation device.

Referring to Figs. 2 and 3, the insert 21 includes a donut shaped resilient member having an annular groove 26 to receive the proximal end 12. Since the resilient member 21 is of resilient material, the edges 27 and 28 of the groove contact the inner and outer surfaces of the tubular member 10 as shown to help effect a seal. Adjacent to the inner periphery of the insert 21 is an annular chamber 29 which is shown in its deflated condition in Fig. 2. A passage way 30 extends from the outer periphery of the insert and is normally maintained in its closed psotion due to the resilience of the material. The surface 32 opposite the annular groove 26 is rounded as shown.

In operation the user M inserts the male genital organ P into the device as shown in Fig. 2. To assist in use of the device the outer surface 14 of the removable end cap 13 is positioned against a rigid object or wall. This allows the user M to apply controlled pressure to the surface 32 to effect a seal between the male genital organ P and the insert 21. If necessary, some pubic hair may be removed to facilitate effecting a seal if lubricant alone is not sufficient. With the male genital organ in the position as shown in Fig. 2, a vacuum is applied to the tube 19 using a suitable vacuum means such as shown in Fig. 1 or by the user drawing air out of the chamber 20 by positioning the end of the tube 19 in the user's mouth and sucking on the tube. Generally a lubricant is necessary to facilitate insertion and removal of the genital organ as well as to aid in effecting a seal.

Prior to applying a vacuum, a syringe 33 having a needle 34 is inserted in the passage way 30 to inflate the annular chamber 29 as shown in Fig. 3. The syringe 33 is used to pump air into the chamber 29 so that when chamber 29 is inflated, the inner peripheral surface 35 engages the male genital organ P to help effect a seal.

Application of a vacuum to the vacuum chamber 20 will induce blood to engorge the male genital member P so that it changes from its flaccid state shown in Fig. 2 to its erect state as shown in Fig. 3. The safety and release valve 22 will protect the user from any harmful effects of excessive vacuum automatically by allowing air into the system if a vacuum greater than that necessary to gain a rigid erection is applied. When a seal is formed between the surface 32 and the user M, the annular chamber 29 need not be inflated until an erection is achieved as shown in Fig. 3. Then the annular chamber 29 is inflated so as to apply a pressure to the base of the male genital member P to restrict the flow of blood out of the member. This maintains the erection for a period of time until the blood gradually flows out of the male genital member P.

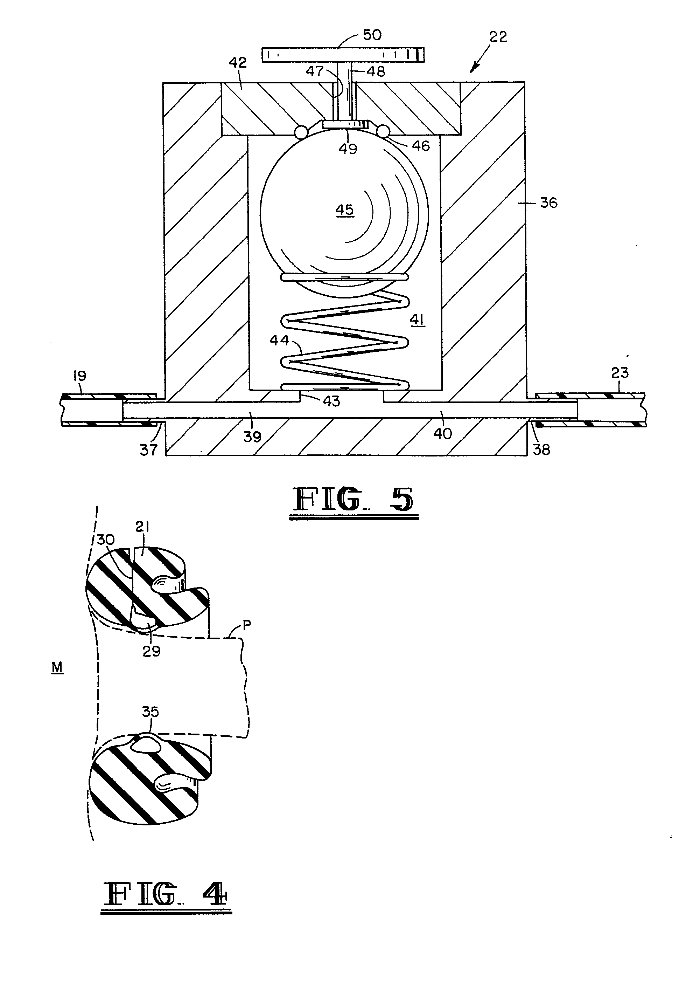

After a sufficient erection is achieved the vacuum is released by the user pressing on button 50 of the safety and release valve 22 and the tubular member 10 is removed from the annular groove 26 which leaves the insert in position on the male genital member as shown in Fig. 4. The inner peripheral surface 35 is shown engaging and applying pressure to the base of the male genital member P after inflation as described above to maintain the male genital member in its erect state. It is important that the pressure applied to the base of the male genital organ not be maintained for more than 30 minutes to avoid harm to the user.

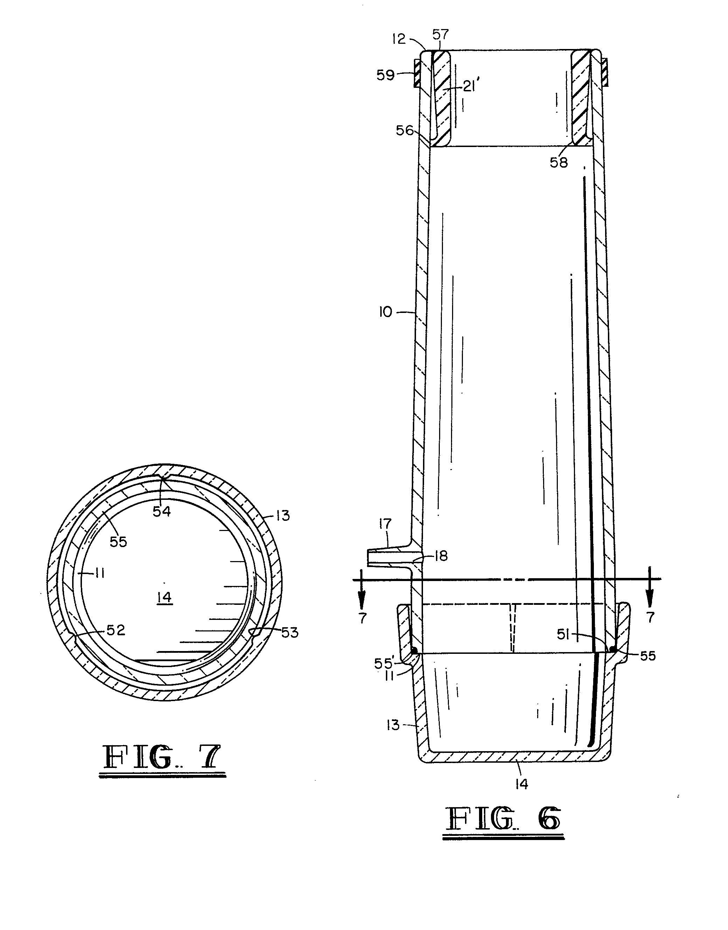

Referring to Fig. 5, the safety and release valve 22 includes a housing 36 which includes a nipple connection 37 and a nipple connection 38 for connecting to the tubes 19 and 23, respectively. A passage way 39 and a passage way 40 communicate with the chamber 41 formed by the housing 36 and end cap member 42. Aperture 43 connects the passage way 39 and 40 to the chamber 41.

Mounted within the chamber 41 is a helical coil spring 44 or other force generating element which engages a ball 45. The ball 45 engages a resilient sealing seat member 46 secured to the end cap member 42. An aperture 47 in the end cap member 42 receives a pin member 48. A configured surface engaging member 49 is secured to the inner end of the pin member 48 and designed to engage the ball valve 45. A press button 50 is secured to the outer end of the pin member 48 to facilitate applying pressure to the press button 50 with one's finger, the pin member 48 and configured member 49 are pushed downwardly to push the ball 45 away from the sealing seat member 46 and vent the chamber and vacuum tubes to the atmosphere to release a vacuum in the chamber. The valve 22 also limits the amount of vacuum applied to the chamber 20 when the vacuum overcomes the force of spring 44 to allow outside air to be supplied to the vacuum source. Spring 44 is selected to automatically provide this safety and releasing feature.

As shown in Fig. 6, the second embodiment includes an insert 21' positioned in the proximal end 10. The insert 21' has a cylindrical inner surface and includes a circular outwardly extending engaging portion 56 which engages the inner surface of the tubular member 10. The member 56 limits the travel of the insert 21' into the tubular member 10 and forms a seal therewith. Ideally it limits the travel so that the end 57 extends at about the same distance so that it mates with the proximal end 12. It may be desirable to have a smaller entrance for the tubular member 10. Varying sized inserts 21' may be used so that users with varying size male genital organs can be custom fit to better effect a seal. In the case of the embodiment shown in Fig. 6 the seal is formed between the surfaces 12 and 57 by pressing the users body against these surfaces with the flat or configured end 14 positioned against a rigid surface or object.

An erection is achieved using the device in Fig. 6 in the same manner as described above in connection with the embodiment in Fig. 1. However, in order to maintain an erection it is necessary to position to cylindrical elastic member 59 on the tubular member 10 adjacent the proximal end 12. In order to maintain the erection the elastic member 59 is slid off of the tubular member 10 so that it applies a pressure at the base of the male genital organ to restrict the flow of blood outwardly and to maintain the erection. The elastic member 59 may be of a single wrap or have multiple wraps around the tubular member 10 and multiple elastic members may be used where necessary. Again it is recommended to never maintain the elastic member 59 on the organ P of the user longer than 30 minutes.

0148586

CLAIMS:

1.    An apparatus for inducing an erection of a male
genital organ, comprising:
      an elongated tubular means (10) having open and closed
ends (12,11) for receiving a male genital organ in the open
end;
      means (17) for connecting a vacuum source (24)with
the tubular means (10) for inducing an erection of the
organ;
      said closed end (11) having removable end closure
and support means (13) for cleaning the tubular means and
which is configured for engaging a support surface with
the tubular means (10) to assist the user in holding and
supporting the tubular means (10) in position on the user's
organ for inducing an erection; and
      said open end (12) having a removable insert means
(21,21') for effecting a seal with the user's organ and the
tubular means (10) to maintain a vacuum in the tubular
means for inducing an erection.

2.    Apparatus as claimed in claim 1 wherein:
      the end of the removable end closure and support
means (13) includes a configured surface for engaging a
support surface.

3.    Apparatus as claimed in claim 1 or 3 wherein:
      the elongated tubular means (10) is circular in
cross-section and tapers from the closed end (11) to the
open end (12).

4.    Apparatus as claimed in any of claims 1 to 3 wherein:
      the removable insert means (21) is made of resilient
material with means (29,30,31) for expanding the resilient
material to form a seal and restrict blood flow out of the
user's organ.

- 10 -

0148586

5. Apparatus as claimed in claim 4, wherein: the means for expanding includes an annular chamber (29) which may be inflated so as to apply a pressure to the base of the user's organ to form a seal to restrict the flow of blood out of the organ and deflated after use.

6. Apparatus as claimed in any preceding claim, wherein: the removable insert means (21, 21') is insertable and removable through the closed end upon disconnecting the removable end closure and support means (13).

7. Apparatus as claimed in any preceding claim, wherein: the connecting means (17) is positioned on the side of the apparatus so that the removable end closure and support means (13) may be engaged and held in position on a support surface.

8. Apparatus as claimed in any preceding claim, wherein: the removable insert means (21) has a sealing portion (35) which reduces the cross sectional size of the open end for effecting the seal with the user's organ.

9. Apparatus as claimed in any preceding claim, wherein: the removable insert means (21,21') includes means (26,56) for engaging the inner surface of the elongated tubular means (10) to prevent removing of the removable insert means (21,21') through the open end and to effect a seal between the removable insert means (21,21') and the inner surface of the elongated tubular means (10).

10. Apparatus as claimed in any preceding claim, wherein: a safety and releasing valve (22) is operatively connected with the tubular means (10) to limit the vacuum applied to the tubular means (10) and for releasing a vacuum in the tubular means.

11.   A method for inducing an erection of a male organ comprising:

inserting a flaccid male genital organ in the open end of an elongated tubular means (10) having open and closed ends (12,11);
connecting a vacuum source (24) with the tubular means (10) for inducing an erection of the organ;
engaging a removable end closure and support means (13) on said closed end (11) of said elongated tubular means (10) with a supporting surface to assist the user in holding and supporting the tubular means (10) in position on the user's organ for inducing an erection; and
applying a controlled and limited vacuum to the tubular means (10) through the connecting means (17) for inducing an erection.

12.   A method as claimed in claim 11, including the step of:

venting the vacuum source (24) to the atmosphere when it exceeds a predetermined level.

13.   A method as claimed in claim 11 or 12, including the step of:

removing the removable end closure and support means (13) and inserting a removable insert means (21,21') into the elongated tubular member (10) to reduce the cross-sectional size of the open end (12) of the elongated tubular member (10) for effecting a seal between the user's organ and the removable insert means (21,21').

0148586

FIG. 1

0148586

FIG. 2

FIG. 3

**FIG. 5**

**FIG. 4**

**FIG. 7**

**FIG. 6**

**European Patent Office**

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | US-A-3 744 486 (WILSON) * Column 2, lines 37-44; figure 1 * | 1 | A 61 F 5/42 |
| Y | US-A-3 820 533 (JONES) | 1 | |
| A | * Claims 1,2; figures 1-3 * | 4,5,8 | |
| A | * Column 2, lines 13-37 * | 11 | |
| A | GB-A-1 497 441 (SUTHERLAND) * Claims 1,7; figure * | 1,10 | |
| A | * Page 1, lines 40-64 | 11 | TECHNICAL FIELDS SEARCHED (Int. Cl.4) A 61 F 5/00 A 61 H 19/00 |
| A | DE-U-7 822 298 (WU, AN-CHUAN) * Claims 1-7; figure * | 1,10 | |
| A | US-A-3 495 589 (CLEMENT) | | |
| D,A | US-A-4 378 008 (OSBON) | | |
| D,A | US-A-2 874 698 (SELL) | | |
| | --- -/- | | |

The present search report has been drawn up for all claims

| Place of search BERLIN | Date of completion of the search 28-02-1985 | Examiner KANAL P K |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

European Patent Office

EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | CH-A- 247 300 (MELDI) | | |
| | --- | | |
| D,A | DE-C- 574 684 (SKORA) | | |
| | ----- | | |

TECHNICAL FIELDS SEARCHED (Int. Cl.4)

The present search report has been drawn up for all claims

| Place of search | Date of completion of the search | Examiner |
|---|---|---|
| BERLIN | 28-02-1985 | KANAL P K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82